# EUROPEAN PATENT APPLICATION

(11) **EP 4 350 705 A1**
(43) Date of publication of application: **10.04.2024**
(21) Application number: 22811596.0
(22) Date of filing: 24.05.2022
(51) Int. Cl.: G16H 30/40, G16H 40/60, G16H 30/20, G16H 50/70, G06T 15/20, G06N 20/00, G06N 3/08, A61B 5/00

(54) **ELECTRONIC DEVICE AND IMAGE PROCESSING METHOD THEREFOR**

(30) Priority: 24.05.2021 KR 20210066144
(71) Applicant: Medit Corp., Seoul 07207 (KR)
(72) Inventor: LEE, Dong Hoon, Seoul 02855 (KR)
(74) Representative: Maiwald GmbH
(86) International application number: PCT/KR2022/007318
(87) International publication number: WO 2022/250403

(57) **Abstract**

Various embodiments disclosed in the present disclosure provide an electronic device comprising: a communication circuit communicatively connected to a three-dimensional scanner; at least one memory configured to store a correlation model constructed by modeling a correlation between a two-dimensional image set regarding oral cavities of subjects and a data set in which a tooth region and a gingival region are identified in each image of the two-dimensional image set according to a machine learning algorithm; and at least one processor, wherein the at least one processor is configured to access a two-dimensional image regarding a target oral cavity or target diagnosis model received from the three-dimensional scanner through the communication circuit, and use the correlation model to identify a tooth region and a gingival region from the two-dimensional image regarding the target oral cavity or target diagnostic model.

## Description

### TECHNICAL FIELD

The present disclosure relates to an electronic device and an image processing method thereof and, more specifically, to an electronic device and an image processing method thereof for processing an image with respect to an oral cavity received from a 3D scanner.

### BACKGROUND

In general, in order to acquire oral information (e.g., information on hard tissues and soft tissues including teeth in an oral cavity) of a patient for a dental diagnosis and treatment, a three-dimensional model of the patient's oral cavity may be acquired by acquiring (or molding) an impression of the patient's oral cavity using a plastic material, and pouring plaster on an impression body molded after the patient's oral cavity.

Recently, in order to acquire oral information of a patient, a three-dimensional scanner that is inserted into the patient's oral cavity and acquires an image for the oral cavity has been used. By scanning the patient's oral cavity by using the three-dimensional scanner, multiple two-dimensional images for the patient's oral cavity may be acquired, and a three-dimensional image for the patient's oral cavity may be constructed using the acquired multiple two-dimensional images. For example, a practitioner may acquire multiple two-dimensional images for the patient's oral cavity by inserting the three-dimensional scanner into the oral cavity of the patient and scanning the patient's teeth, gingiva, and/or soft tissues. Thereafter, by applying a three-dimensional modeling technology, a three-dimensional image for the patient's oral cavity may be constructed using the two-dimensional images for the patient's oral cavity.

### SUMMARY

Various embodiments disclosed in the present disclosure provide a technology for identifying various regions included in a two-dimensional image for a patient's oral cavity or a diagnostic model of the oral cavity through machine learning so that a user may easily configure a region of interest to reconstruct a three-dimensional image for the oral cavity.

Various embodiments disclosed in the present disclosure provide an electronic device comprising: a communication circuit communicatively connected to a three-dimensional scanner; at least one memory configured to store a correlation model constructed by modeling a correlation between a two-dimensional image set regarding oral cavities of subjects and a data set in which a tooth region and a gingival region are identified in each image of the two-dimensional image set according to a machine learning algorithm; and at least one processor, wherein the at least one processor is configured to access a two-dimensional image regarding a target oral cavity or target diagnosis model received from the three-dimensional scanner through the communication circuit, and use the correlation model to identify a tooth region and a gingival region from the two-dimensional image regarding the target oral cavity or target diagnostic model.

Various embodiments disclosed in the present disclosure provide an image processing method of an electronic device, the method comprising: accessing a two-dimensional image regarding a target oral cavity or target diagnosis model received from a three-dimensional scanner, and using a correlation model to identify a tooth region and a gingival region from the two-dimensional image regarding the target oral cavity or target diagnostic model, wherein the correlation model is a correlation model constructed by modeling a correlation between a two-dimensional image set regarding oral cavities of subjects and a data set in which a tooth region and a gingival region are identified in each image of the two-dimensional image set according to a machine learning algorithm.

Various embodiments disclosed in the present disclosure provide an electronic device for training a machine learning model used for identifying a tooth region and a gingival region, the electronic device comprising: at least one memory configured to store a two-dimensional image set regarding oral cavities of subjects or oral cavity diagnosis model and a data set in which a tooth region and a gingival region are identified in each image of the two-dimensional image set, and at least one processor, wherein the at least one processor is configured to use the two-dimensional image set of the oral cavities of the subjects as input data and the data set in which the tooth region and the gingival region are identified in each image of the two-dimensional image set as output data, so as to train a machine learning model.

According to various embodiments of the present disclosure, it is possible to identify a tooth region and a gingiva region from two-dimensional images for the oral cavity or a diagnostic model of the oral cavity by using a correlation model constructed through machine learning and to select at least one region from among regions identified by a user to generate a three-dimensional image for the oral cavity and diagnosis model so that the user may conveniently and quickly generate a three-dimensional image for a desired region.

According to various embodiments of the present disclosure, it is possible to identify the tooth region and the gingiva region from the two-dimensional images for the oral cavity or the diagnostic model of the oral cavity by using the correlation model and generate a three-dimensional image for the oral cavity or the diagnostic model of the oral cavity corresponding to the at least one region selected from among the identified regions, so that the amount of data required to generate the three-dimensional image may be reduced, and the time and resources required to generate the three-dimensional image may be reduced.

### BRIEF DESCRIPTION OF DRAWINGS

FIG. 1 is a view illustrating an operation of acquiring an image for a patient's oral cavity by using a three-dimensional scanner according to various embodiments of the present disclosure.
FIG. 2A is a block view illustrating an electronic device and a three-dimensional scanner according to various embodiments of the present disclosure. FIG. 2B is a perspective view illustrating a three-dimensional scanner according to various embodiments of the present disclosure.
FIG. 3 is a view illustrating a method for generating a three-dimensional image for an oral cavity according to various embodiments of the present disclosure.
FIGS. 4A and 4B are views illustrating performing a masking operation on a two-dimensional image for an oral cavity according to various embodiments of the present disclosure.
FIG. 5 is a view illustrating a method for constructing a correlation model according to various embodiments of the present disclosure.
FIGS. 6A and 6B are views illustrating a method for using a correlation model according to various embodiments of the present disclosure.
FIG. 7 is an operational flowchart of an electronic device according to various embodiments of the present disclosure.
FIG. 8 is an operational flowchart of an electronic device according to various embodiments of the present disclosure.
FIGS. 9A to 9C are views illustrating a three-dimensional image for a target oral cavity according to various embodiments of the present disclosure.

### DETAILED DESCRIPTION

Embodiments of the present disclosure are illustrated for describing the technical spirit of the present disclosure. The scope of the claims according to the present disclosure is not limited to the embodiments described below or to the detailed descriptions of these embodiments.

All technical and scientific terms used in the present disclosure have meanings commonly understood by those of ordinary skill in the art to which the present disclosure belongs, unless otherwise defined. All terms used in the present disclosure are selected for more clearly describing the present disclosure and are not selected to limit the scope of rights according to the present disclosure.

As used in the present disclosure, expressions such as "comprising," "including," "having," and the like are to be understood as open-ended terms having the possibility of encompassing other embodiments, unless otherwise mentioned in the phrase or sentence containing such expressions.

The singular form, when used in the present disclosure may include a meaning of a plurality, unless otherwise mentioned, and the same is applied to a singular expression stated in the claims. The expressions "first," "second," and the like used in the present disclosure, are intended to distinguish between multiple elements, and are not intended to limit the sequence or importance of the corresponding elements.

The term "unit" used in the present disclosure means a software component or hardware component, such as a field-programmable gate array (FPGA) and an application specific integrated circuit (ASIC). However, a "unit" is not limited to hardware and software. A "unit" may be configured to be in an addressable storage medium or may be configured to run on one or more processors. Therefore, as an example, a "unit" may include components, such as software components, object-oriented software components, class components, and task components, as well as processors, functions, attributes, procedures, subroutines, segments of program codes, drivers, firmware, micro-codes, circuits, data, databases, data structures, tables, arrays, and variables. Functions provided in components and "units" may be combined into a smaller number of components and "units" or subdivided into additional components and "units."

The expression "on the basis of' or "based on" used in the present disclosure is used to describe one or more factors that influence a decision, an activity of judgment, or an operation described in a phrase or sentence including the relevant expression, and this expression does not exclude an additional factor influencing the decision, the activity of judgment, or the operation.

It will be understood that when a component is referred to as being "coupled" or "connected" to another component in the present disclosure, it may be directly coupled or connected to the other component, or intervening components may be present therebetween.

In the present disclosure, artificial intelligence (AI) means a technology that imitates human learning ability, reasoning ability, and perception ability and implements them with a computer, and may include concepts of machine learning and symbolic logic. Machine learning (ML) may include an algorithm technology that classifies or learns features of input data by itself. Artificial intelligence technology is a machine learning algorithm that analyzes input data, learns a result of the analysis, and may make judgments or predictions on the basis of a result of the learning. In addition, technologies that use machine learning algorithms to mimic cognitive and judgmental functions of the human brain may also be understood as the category of artificial intelligence. For example, technical fields of linguistic understanding, visual understanding, inference/prediction, knowledge expression, and motion control may be included.

In the present disclosure, machine learning may refer to a process of training a neural network model by using experience of processing data. It may refer to computer software improving its own data processing capabilities through machine learning. A neural network model is constructed by modeling a correlation between data, and the correlation may be expressed by multiple parameters. The neural network model derives a correlation between data by extracting and analyzing features from given data, and optimizing the parameters of the neural network model by repeating the process may be referred to as machine learning. For example, the neural network model may learn a mapping (correlation) between an input and an output with respect to data given in a form of an input/output pair. Alternatively, even when only input data is given, the neural network model may derive a regularity between given data to learn a relationship therebetween.

In the present disclosure, an artificial intelligence learning model, a machine learning model, or a neural network model may be designed to implement a human brain structure on a computer, and may include multiple network nodes that simulate neurons of a human neural network and have weights. The multiple network nodes may have a connection relationship therebetween by simulating synaptic activities of neurons that transmit and receive a signal through synapses. In the artificial intelligence learning model, the multiple network nodes may transmit and receive data according to a convolution connection relationship while being located in layers of different depths. The artificial intelligence learning model may include, for example, an artificial neural network model, a convolution neural network model, and the like.

Hereinafter, embodiments of the present disclosure will be described with reference to the accompanying drawings. Throughout the accompanying drawings, identical or corresponding elements are given the same reference numerals. Furthermore, repeated description of the same or corresponding components may be omitted in the following description of embodiments. However, omission of a description of components is not intended to mean exclusion of the components from the embodiments.

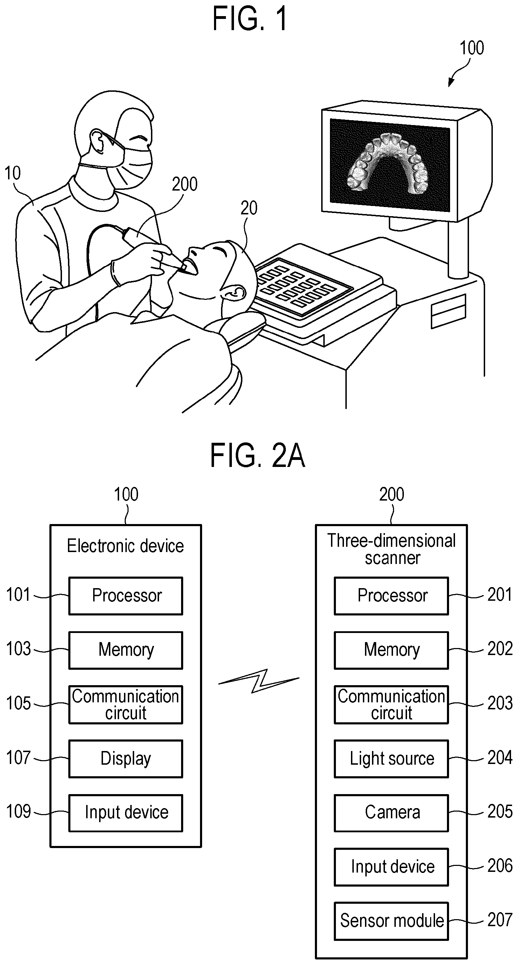

FIG. 1 is a view illustrating an operation of acquiring an image for a patient's oral cavity by using a three-dimensional scanner 200 according to various embodiments of the present disclosure. According to various embodiments, the three-dimensional scanner 200 may correspond to a dental medical device for acquiring an image for the oral cavity of a subject 20. For example, the three-dimensional scanner 200 may include an intraoral scanner. As shown in FIG. 1, a user 10 (e.g., a dentist or a dental hygienist) may acquire an image for an oral cavity of the subject 20 (e.g., a patient) using the three-dimensional scanner 200. As another example, the user 10 may acquire an image for the oral cavity of the subject 20 from a diagnosis model (e.g., a plaster model or an impression model) molded after the shape of the oral cavity of the subject 20. Hereinafter, for convenience of explanation, it will be described that an image for the oral cavity of the subject 20 is acquired by scanning the oral cavity of the subject 20, but is not limited thereto, and it is possible to acquire an image for other parts of the subject 20 (e.g., the ear of the subject). The three-dimensional scanner 200 may have a shape capable of being inserted in and drawn out of the oral cavity, and may be a handheld scanner in which the user 10 may freely adjust a scanning distance and a scanning angle.

The three-dimensional scanner 200 according to various embodiments may acquire an image for the oral cavity by being inserted into the oral cavity of the subject 20 and scanning the oral cavity in a non-contact manner. The image for the oral cavity may include at least one tooth, a gingiva, and an artificial structure insertable into the oral cavity (e.g., an orthodontic device including a bracket and a wire, an implant, a denture, and an orthodontic aid inserted into the oral cavity). The three-dimensional scanner 200 may emit light to the oral cavity (e.g., at least one tooth or gingiva of the subject 20) of the subject 20 using a light source (or projector), and may receive light reflected from the oral cavity of the subject 20 through a camera (or, at least one image sensor). According to another embodiment, the three-dimensional scanner 200 may obtain an image for an oral cavity diagnostic model by scanning the oral cavity diagnostic model. When the diagnostic model of the oral cavity is a diagnostic model that is molded after the shape of the oral cavity of the subject 20, the image for the oral diagnostic model may be an image for the oral cavity of the subject. Hereinafter, for convenience of explanation, a case in which an image for the oral cavity is acquired by scanning the inside of the oral cavity of the subject 20 is assumed, but is not limited thereto.

The three-dimensional scanner 200 according to various embodiments may acquire a surface image for the oral cavity of the subject 20 as a 2D image based on information received through a camera. The surface image for the oral cavity of the subject 20 may include at least one tooth, gingiva, artificial structure, and at least one of cheek, tongue, or lip of the subject 20. The surface image for the oral cavity of the subject 20 may be a two-dimensional image.

The two-dimensional images for the oral cavity acquired by the three-dimensional scanner 200 according to various embodiments may be transmitted to an electronic device 100 connected through a wired or wireless communication network. The electronic device 100 may include a computer device or a portable communication device. The electronic device 100 may generate a three-dimensional image (or three-dimensional oral cavity image or a three-dimensional oral cavity model) for the oral cavity, which represents the oral cavity three-dimensionally on the basis of the two-dimensional images for the oral cavity received from the three-dimensional scanner 200. The electronic device 100 may generate a three-dimensional image for the oral cavity by three-dimensionally modeling the internal structure of the oral cavity based on the received two-dimensional images for the oral cavity.

The three-dimensional scanner 200 according to another embodiment may scan the oral cavity of the subject 20 to acquire two-dimensional images for the oral cavity, and generate a three-dimensional image for the oral cavity based on the acquired two-dimensional images for the oral cavity, and transmit the generated three-dimensional image for the oral cavity to the electronic device 100.

According to various embodiments, the electronic device 100 may be communicatively connected to a cloud server (not shown). In this case, the electronic device 100 may transmit the two-dimensional images for the oral cavity of the subject 20 or the three-dimensional image for the oral cavity to the cloud server, and the cloud server may store the two-dimensional images for the oral cavity of the subject 20 or the three-dimensional image for the oral cavity received from the electronic device 100.

According to another embodiment, a table scanner (not shown) fixed to a specific position for use may be used as the three-dimensional scanner in addition to the handheld scanner inserted into the oral cavity of the subject 20 for use. The table scanner may generate a three-dimensional image for the oral cavity diagnostic model by scanning the oral cavity diagnostic model. In this case, since a light source (or projector) and a camera of the table scanner are fixed, the user may scan the oral diagnosis model while moving the oral diagnosis model.

FIG. 2A is a block view illustrating an electronic device 100 and a three-dimensional scanner 200 according to various embodiments of the present disclosure. The electronic device 100 and the three-dimensional scanner 200 may be communicatively connected to each other through a wired or wireless communication network, and may transmit and receive various data to and from each other.

The three-dimensional scanner 200 according to various embodiments may include a processor 201, a memory 202, a communication circuit 203, a light source 204, a camera 205, an input device 206, and/or a sensor module 207. At least one of components included in the three-dimensional scanner 200 may be omitted or another component may be added to the three-dimensional scanner 200. Additionally or alternatively, some of the components may be integrated and implemented, or implemented as a singular entity or plural entities. At least some of the components in the three-dimensional scanner 200 may be connected to each other through a bus, a general purpose input/output (GPIO), a serial peripheral interface (SPI) or a mobile industry processor interface (MIPI), and transmit and receive data and/or a signal.

The processor 201 of the three-dimensional scanner 200 according to various embodiments corresponds to a component capable of performing calculation or data processing for control and/or communication of each component of the three-dimensional scanner 200 and may be operatively connected to the components of the three-dimensional scanner 200. The processor 201 may load a command or data received from other components of the three-dimensional scanner 200 into the memory 202, process the command or data stored in the memory 202, and store result data. The memory 202 of the three-dimensional scanner 200 according to various embodiments may store instructions for an operation of the above-described processor 201.

According to various embodiments, the communication circuit 203 of the three-dimensional scanner 200 may establish a wired or wireless communication channel with an external device (e.g., the electronic device 100) and transmit or receive various data to or from the external device. According to an embodiment, the communication circuit 203 may include at least one port for connection with the external device through a wired cable to communicate with the external device by wire. In this case, the communication circuit 203 may perform communication in a wired manner with an external device connected through at least one port. According to an embodiment, the communication circuit 203 may include a cellular communication module and may be configured to be connected to a cellular network (e.g., 3G, LTE, 5G, Wibro, or WiMAX). According to various embodiments, the communication circuit 203 may include a near field communication module to transmit or receive data to or from an external device using near field communication (e.g., Wi-Fi, Bluetooth, Bluetooth Low Energy (BLE), and UWB), but is not limited thereto. According to an embodiment, the communication circuit 203 may include a non-contact communication module for non-contact communication. The non-contact communication may include, for example, at least one non-contact type proximity communication technology such as near field communication (NFC), radio frequency identification (RFID) communication, or magnetic secure transmission (MST) communication.

The light source 204 of the three-dimensional scanner 200 according to various embodiments may emit light toward the oral cavity of the subject 20. For example, the light emitted from the light source 204 may include structured light having a predetermined pattern (e.g., a stripe pattern in which straight lines of different colors continuously appear). The structured light pattern may be generated using, for example, a pattern mask or a digital micro-mirror device (DMD), but is not limited thereto. The camera 205 of the three-dimensional scanner 200 according to various embodiments may acquire an image for the oral cavity of the subject 20 by receiving reflected light reflected by the oral cavity of the subject 20. The camera 205 may include, for example, a left camera corresponding to a left eye field of view and a right camera corresponding to a right eye field of view to construct a three-dimensional image according to an optical triangulation method. The camera 205 may include at least one image sensor such as a CCD sensor or a CMOS sensor.

The input device 206 of the three-dimensional scanner 200 according to various embodiments may receive a user input for controlling the three-dimensional scanner 200. The input device 206 may include a button for receiving a push manipulation of the user 10, a touch panel for detecting a touch of the user 10, and a voice recognition device including a microphone. For example, the user 10 may control scanning start or stop by using the input device 206.

The sensor module 207 of the three-dimensional scanner 200 according to various embodiments may detect an operation state of the three-dimensional scanner 200 or an external environmental state (e.g., a user's motion), and generate an electrical signal corresponding to the detected state. The sensor module 207 may include, for example, at least one of a gyro sensor, an acceleration sensor, a gesture sensor, a proximity sensor, or an infrared sensor. For example, the user 10 may control scanning start or stop by using the sensor module 207. For example, when the user 10 holds the three-dimensional scanner 200 in his or her hand and moves the same, the three-dimensional scanner 200 may control, when an angular velocity measured through the sensor module 207 exceeds a predetermined threshold value, the processor 201 to start a scanning operation.

According to an embodiment, the three-dimensional scanner 200 may start scanning when receiving a user's input for starting scanning through the input device 206 of the three-dimensional scanner 200 or the input device 206 of the electronic device 100, or according to processing by the processor 201 of the three-dimensional scanner 200 or the processor 201 of the electronic device 100. When the user 10 scans the inside of the oral cavity of the subject 20 through the three-dimensional scanner 200, the three-dimensional scanner 200 may generate two-dimensional images for the oral cavity of the subject 20, and may transmit the two-dimensional images for the oral cavity of the subject 20 to the electronic device 100 in real time. The electronic device 100 may display the received two-dimensional images for the oral cavity of the subject 20 through the display. Furthermore, the electronic device 100 may generate (construct) a three-dimensional image for the oral cavity of the subject 20 based on the two-dimensional images for the oral cavity of the subject 20, and display the three-dimensional image for the oral cavity through the display. The electronic device 100 may display the 3D image being generated through the display in real time.

The electronic device 100 according to various embodiments may include at least one processor 101, at least one memory 103, a communication circuit 105, a display 107, and/or an input device 109. At least one of the components included in the electronic device 100 may be omitted or another component may be added to the electronic device 100. Additionally or alternatively, some of the components may be integrated and implemented, or implemented as a singular or plural entity. At least some of the components in the electronic device 100 may be connected to each other through a bus, a general purpose input/output (GPIO), a serial peripheral interface (SPI) or a mobile industry processor interface (MIPI), and transmit and receive data and/or a signal.

According to various embodiments, the at least one processor 101 of the electronic device 100 may correspond to a component capable of performing calculation or data processing for control and/or communication of each component (e.g., the memory 103) of the electronic device 100. The at least one processor 101 may be operatively connected, for example, to components of the electronic device 100. The at least one processor 101 may load a command or data received from other components of the electronic device 100 into the at least one memory 103, process the command or data stored in the at least one memory 103, and store result data.

According to various embodiments, the at least one memory 103 of the electronic device 100 may store instructions for an operation of the at least one processor 101. The at least one memory 103 may store correlation models constructed according to a machine learning algorithm. The at least one memory 103 may store data (e.g., two-dimensional images for the oral cavity acquired through the oral cavity scan) received from the three-dimensional scanner 200.

According to various embodiments, the communication circuit 105 of the electronic device 100 may establish a wired or wireless communication channel with an external device (e.g., the three-dimensional scanner 200 or the cloud server) and transmit or receive various data to or from the external device. According to an embodiment, the communication circuit 105 may include at least one port for connection with the external device through a wired cable to communicate with the external device by wire. In this case, the communication circuit 105 may perform communication in a wired manner with an external device connected through at least one port. According to an embodiment, the communication circuit 105 may include a cellular communication module and may be configured to be connected to a cellular network (e.g., 3G, LTE, 5G, Wibro, or WiMAX). According to various embodiments, the communication circuit 105 may include a near field communication module to transmit or receive data to or from an external device using near field communication (e.g., Wi-Fi, Bluetooth, Bluetooth Low Energy (BLE), and UWB), but is not limited thereto. According to an embodiment, the communication circuit 105 may include a non-contact communication module for non-contact communication. The non-contact communication may include, for example, at least one non-contact type proximity communication technology such as near field communication (NFC) communication, radio frequency identification (RFID) communication, or magnetic secure transmission (MST) communication.

According to various embodiments, the display 107 of the electronic device 100 may display various screens based on control of the processor 101. The processor 101 may display, through the display 107, two-dimensional images for the oral cavity of the subject 20 received from the three-dimensional scanner 200 and/or a three-dimensional image for the oral cavity, in which an internal structure of the oral cavity is three-dimensionally modeled. For example, the two-dimensional images and/or the three-dimensional image for the oral cavity may be displayed through a specific application. In this case, the user 10 may edit, save, and delete the two-dimensional images and/or the three-dimensional image for the oral cavity.

The input device 109 of the electronic device 100 according to various embodiments may receive a command or data to be used for a component (e.g., the at least on processor 101) of the electronic device 100 from the outside (e.g., the user) of the electronic device 100. The input device 109 may include, for example, a microphone, a mouse, or a keyboard. According to an embodiment, the input device 109 may be combined with the display 107 and implemented in a form of a touch sensor panel capable of recognizing contact or proximity of various external objects.

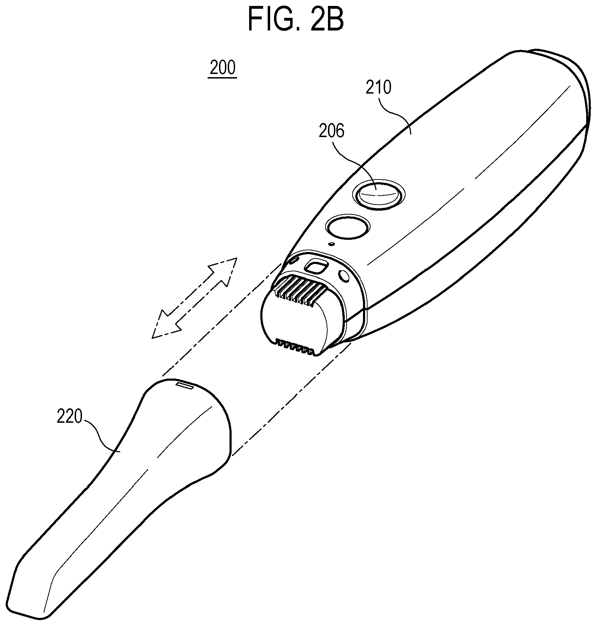

FIG. 2B is a perspective view illustrating a three-dimensional scanner 200 according to various embodiments. The three-dimensional scanner 200 according to various embodiments may include a body 210 and a probe tip 220. The body 210 of the three-dimensional scanner 200 may have a shape that is easy for the user 10 to grip and use. The probe tip 220 may have a shape that facilitates insertion into and withdrawal from the oral cavity of the subject 20. Furthermore, the body 210 may be coupled to and separated from the probe tip 220. The body 210 may have the components of the three-dimensional scanner 200 described in FIG. 2A disposed therein. An opening may be disposed at one end of the body 210 so that light output from the light source 204 may be emitted to the subject 20. The light emitted through the opening may be reflected by the subject 20 and introduced again through the opening. The reflected light introduced through the opening may be captured by a camera to generate an image for the subject 20. The user 10 may start scanning by using the input device 206 (e.g., a button) of the three-dimensional scanner 200. For example, when the user 10 touches or presses the input device 206, light may be emitted from the light source 204 to the subject 20.

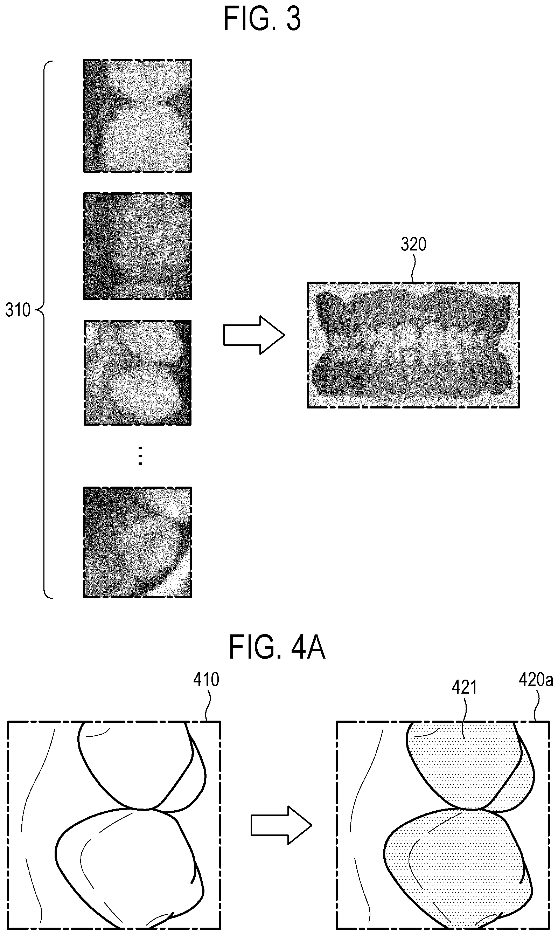

FIG. 3 is a view illustrating a method for generating a three-dimensional image 320 for an oral cavity according to various embodiments. The user 10 may scan the inside of the oral cavity of the subject 20 while moving the three-dimensional scanner 200, and in this case, the three-dimensional scanner 200 may acquire multiple two-dimensional images 310 for the oral cavity of the subject 20. For example, the three-dimensional scanner 200 may acquire a two-dimensional image for a region including a front tooth of the subject 20, a two-dimensional image for a region including a molar tooth of the subject 20, and the like. The three-dimensional scanner 200 may transmit the acquired multiple two-dimensional images 310 to the electronic device 100. According to another embodiment, the user 10 may scan a diagnostic model of the oral cavity while moving the three-dimensional scanner 200, or acquire multiple two-dimensional images for the diagnostic model of the oral cavity. Hereinafter, for convenience of explanation, a case in which an image for the oral cavity of the subject 20 is acquired by scanning the inside of the oral cavity of the subject 20 is assumed, but is not limited thereto.

The electronic device 100 according to various embodiments may convert each of the multiple two-dimensional images 310 of the oral cavity of the subject 20 into a set of multiple points having three-dimensional coordinate values. For example, the electronic device 100 may convert each of the multiple two-dimensional images 310 into a point cloud, which is a set of data points having three-dimensional coordinate values. For example, the point cloud set corresponding to three-dimensional coordinate values based on the multiple two-dimensional images 310 may be stored as raw data for the oral cavity of the subject 20. The electronic device 100 may complete the entire tooth model by aligning the point cloud, which is a set of data points having three-dimensional coordinate values.

The electronic device 100 according to various embodiments may reconfigure (reconstruct) a three-dimensional image for the oral cavity. For example, by merging point cloud sets stored as raw data by using a Poisson algorithm, the electronic device 100 may reconfigure multiple points and convert the multiple points into a closed three-dimensional surface to reconfigure the three-dimensional image 320 of the oral cavity of the subject 20.

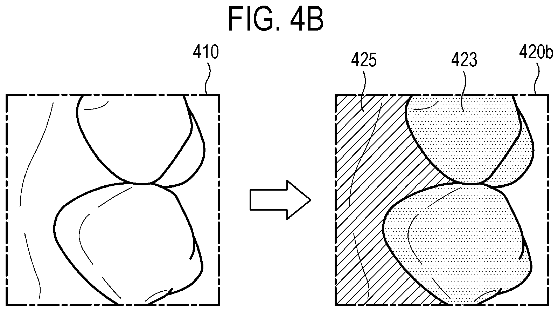

FIGS. 4A and 4B are views illustrating performing a masking operation on a two-dimensional image for an oral cavity according to various embodiments of the present disclosure. The electronic device 100 according to various embodiments may generate (construct) a correlation model for identifying (distinguishing) a tooth region and/or a gingival region in a two-dimensional image for the oral cavity. In order to train a machine learning model used for segmentation of the tooth region and/or the gingival region, the electronic device 100 may use, as learning (training data), a two-dimensional image set of the oral cavity of the subject as well as a data set in which the tooth region and the gingival region are identified in each image of the two-dimensional image set. The two-dimensional image for the oral cavity may include a two-dimensional image for the oral cavity of the subject 20 acquired by scanning the subject 20 and may include a two-dimensional image for the diagnosis model acquired by scanning the diagnosis model of the oral cavity molded after the shape of the oral cavity of the subject 20.

According to an embodiment, the data set in which the tooth region and the gingival region are identified in each image of the two-dimensional image set may have a form of an image. In this case, the data set may be, for example, multiple two-dimensional images in which each of the tooth region and the gingival region is masked in each image of the two-dimensional image set. According to another embodiment, the data set in which the tooth region and the gingival region are identified in each image of the two-dimensional image set may have a form of metadata. In this case, the data set may be, for example, metadata indicating each of the tooth region and the gingival region in each image of the two-dimensional image set.

FIGS. 4A and 4B are exemplary views illustrating a procedure of generating learning data required for training a machine learning model. According to various embodiments, the electronic device 100 may mask only a predetermined region of a two-dimensional image 410 of the oral cavity. According to an embodiment, as shown in FIG. 4A, the electronic device 100 may mask only a tooth region 421 in the two-dimensional image 410 of the oral cavity and may not mask remaining regions such as the gingival region and a soft tissue region (e.g., a cheek region, a tongue region, and the like). Here, masking may be an operation by which a predetermined region is distinguished from other regions. For example, when receiving a user input for masking only the tooth region 421 in the two-dimensional image 410 of the oral cavity from the user, the electronic device 100 may acquire an image 420a, in which the tooth region 421 is masked, in response to the user input. For example, as in the masked image 420a, the tooth region 421 may be displayed to be distinguished from other regions by displaying the tooth region as a dot region. For example, the tooth region 421 may also be displayed to be distinguished from other regions by displaying the tooth region as a red region.

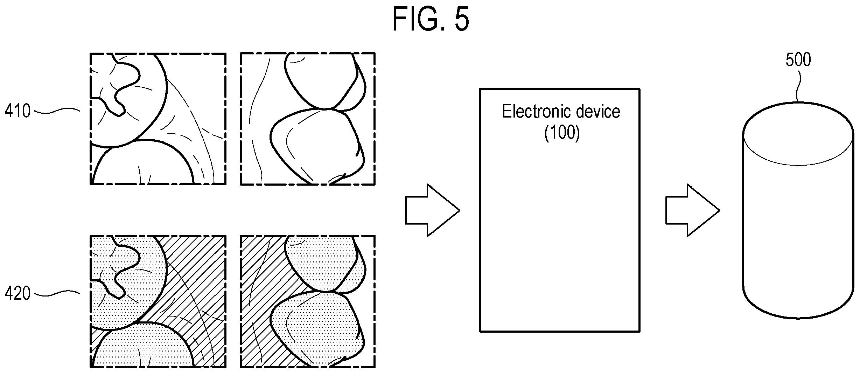

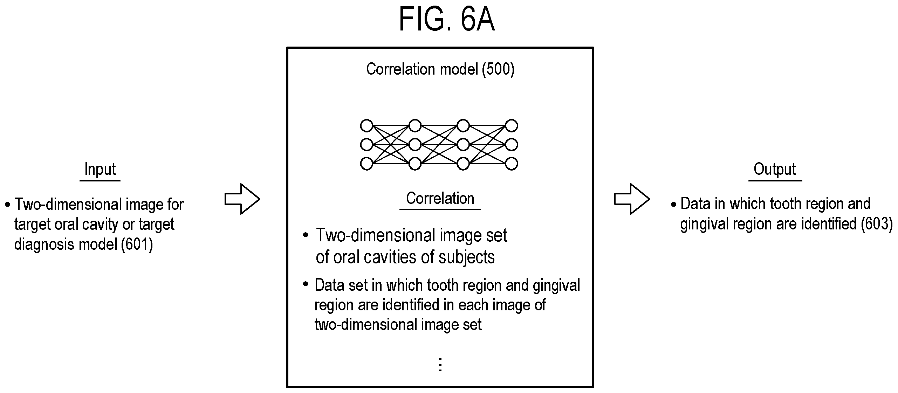

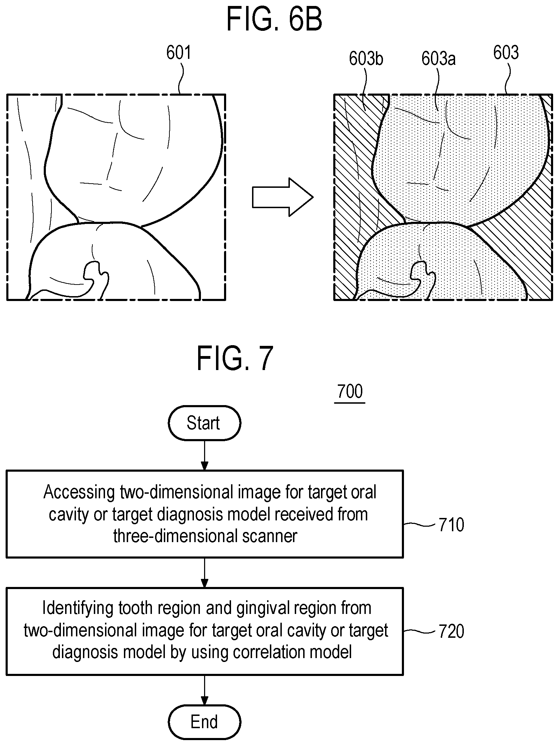

The electronic device 100 may use a two-dimensional image set regarding oral cavities of subjects as input data and a data set in which the tooth region is identified in each image of the two-dimensional image set as output data, so as to train the machine learning model. Here, the data set in which the tooth region is identified in each image of the two-dimensional image set may include, for example, a two-dimensional image 420a in which the tooth region 421 is masked in the two-dimensional image 410. According to the present embodiment, the electronic device 100 may construct a correlation model acquired by modeling a correlation between the two-dimensional image set regarding oral cavities of subjects and the data set in which the tooth region is identified in each image of the two-dimensional image set. A detailed method of constructing and using the correlation model will be described below with reference to FIGS. 5, 6A, and 6B.

According to various embodiments, the electronic device 100 may mask only each of multiple regions in the two-dimensional image 410 of the oral cavity. According to an embodiment, as shown in FIG. 4B, the electronic device 100 may mask each of a tooth region 423 and a gingival region 425 in the two-dimensional image 410 of the oral cavity and may not mask a soft tissue region corresponding to a remaining region. For example, when receiving a user input for masking each of the tooth region 423 and the gingival region 425 in the two-dimensional image 410 of the oral cavity from the user, the electronic device 100 may acquire an image 420b, in which each of the tooth region 423 and the gingival region 425 is masked, in response to the user input. For example, as in the masked image 420b, the electronic device 100 may display the tooth region 423 as a dot region and the gingival region 425 as a hatched region so as to be distinguished from other regions. For example, the electronic device 100 may display the tooth region 423 as a red region and the gingival region 425 as a blue region so as to be distinguished from other regions.

The electronic device 100 may use a two-dimensional image set regarding oral cavities of subjects as input data and a data set in which the tooth region and the gingival region are identified in each image of the two-dimensional image set as output data, so as to train the machine learning model. Here, the data set in which the tooth region and the gingival region are identified in each image of the two-dimensional image set may include, for example, a two-dimensional image 420b in which each of the tooth region 423 and the gingival region 425 is masked in the two-dimensional image 410. According to the present embodiment, the electronic device 100 may construct a correlation model acquired by modeling a correlation between the two-dimensional image set regarding oral cavities of subjects and the data set in which the tooth region and the gingival region are identified in each image of the two-dimensional image set.

According to an embodiment, the electronic device 100 may separately mask each of multiple regions included in the two-dimensional image for the oral cavity. For example, the user may mask each of a tooth region, a gingival region, a cheek region, a lip region, a tongue region, and a metal region so as to be identifiable. For example, when receiving a user input for masking each of the tooth region, the gingival region, the cheek region, the lip region, the tongue region, and the metal region in the two-dimensional image for the oral cavity from the user, the electronic device 100 may acquire an image (not shown), in which the above-described regions are masked, in response to the user input. According to the present embodiment, the electronic device 100 may construct a correlation model acquired by modeling a correlation between the two-dimensional image set regarding oral cavities of subjects and the data set in which each of the tooth region, the gingival region, the cheek region, the lip region, the tongue region, and the metal region is identified in each image of the two-dimensional image set.

The method of masking the two-dimensional image for the oral cavity is not limited to the above-described embodiments, and at least one of multiple regions included in the two-dimensional image may be masked so as to be identified as necessary.

FIG. 5 is a view illustrating a method for constructing a correlation model according to various embodiments of the present disclosure. Hereinafter, for convenience of explanation, constructing a correlation model 500 for identifying each of the tooth region and the gingival region will be described, but the present disclosure is not limited thereto, and a correlation model for identifying each of one or more regions among multiple regions included in the two-dimensional image may be constructed as necessary.

Referring to FIG. 5, the electronic device 100 according to various embodiments may construct a correlation model 500 by using the two-dimensional image set 410 regarding oral cavities of subjects and the data set 420 in which the tooth region and the gingival region are identified in each image of the two-dimensional image set 410. The data set 420 in which the tooth region and the gingival region are identified in each image of the two-dimensional image set 410 may include, for example, multiple two-dimensional images in which each of tooth region and the gingival region is masked in each image of the two-dimensional image set 410. Each of the two-dimensional image sets 410 of the oral cavities of the subjects may be combined with corresponding data of the data set 420 in which the tooth region and the gingival region are identified. The two-dimensional image set 410 of the oral cavities of subjects may include, for example, multiple two-dimensional images for the oral cavities acquired by scanning the oral cavity of the subject 20 and may include multiple two-dimensional images for the diagnosis model for oral cavity acquired by scanning the diagnosis model molded after the shape of the oral cavity of the subject 20.

The correlation model 500 according to various embodiments may correspond to a correlation model constructed by modeling a correlation between the two-dimensional image set 410 regarding oral cavities of subjects and the data set 420 in which the tooth region and the gingival region are identified in each image of the two-dimensional image set 410. The machine learning algorithm may include, for example, one of a deep neural network, a recurrent neural network, a convolutional neural network, a machine learning model for classification-regression analysis, or a reinforcement learning model.

The electronic device 100 may use the two-dimensional image set 410 regarding oral cavities of subjects as input data and the data set 420 in which the tooth region and the gingival region are identified in each image of the two-dimensional image set 410 as output data, so as to train the machine learning model. The electronic device 100 may construct the correlation model 500 acquired by modeling a correlation between the two-dimensional image set 410 regarding oral cavities of subjects and the data set 420 in which the tooth region and the gingival region are identified in each image of the two-dimensional image set 410 through the aforementioned machine learning process.

The correlation model 500 may correspond to a correlation model machine-trained to extract at least one feature of texture, density, color, tooth shape, gingival shape, and oral cavity shape of the tooth region and the gingival region identified in each image of the two-dimensional image set 410, and derive a correlation between the two-dimensional image set 410 and the data set 420 by using the extracted at least one feature. A method of acquiring data on the tooth region and the gingival region from the two-dimensional image for the oral cavity using the constructed correlation model 500 will be described below.

In the present figure, the description is given of an embodiment in which multiple two-dimensional images, in which each of the tooth region and the gingival region is masked in each image of the two-dimensional image set, are used as training data to construct the correlation model, but the present disclosure is not limited thereto, and a correlation model may be constructed using various data sets, for example, meta data indicating each of the tooth region and the gingival region in each image of the two-dimensional image set as training data.

FIGS. 6A and 6B are views illustrating a method for using a constructed correlation model 500 according to various embodiments of the present disclosure. As described above, the correlation model 500 may be constructed according to a machine learning algorithm.

Referring to FIGS. 6A and 6B, the electronic device 100 according to various embodiments may acquire data in which the tooth region and the gingival region are identified from a two-dimensional image 601 of an oral cavity (hereinafter, referred to as a target oral cavity) of a target subject by using the constructed correlation model 500. For example, the electronic device 100 may access the two-dimensional image 601 of the target oral cavity received from the three-dimensional scanner 200, and input the two-dimensional image 601 of the target oral cavity into the correlation model 500 so as to derive data in which the tooth region and the gingival region are identified. The data in which the tooth region and the gingival region are identified may represent, for example, an image in which the tooth region 603a and the gingival region 603b are masked on the input two-dimensional image 601 of the target oral cavity. The data in which the tooth region and the gingival region are identified may represent, for example, metadata indicating positions of the tooth region and the gingival region on the input two-dimensional image 601 of the target oral cavity. Accordingly, the position of each of the tooth region and the gingival region may be identified from the two-dimensional image 601 of the target oral cavity received from the three-dimensional scanner 200 by using the constructed correlation model 500. The data in which the tooth region and the gingival region are identified in the two-dimensional image for the target oral cavity by using the correlation model 500 may be used for generating a three-dimensional image for the target oral cavity.

According to another embodiment, by using the constructed correlation model 500, the electronic device 100 may acquire data in which the tooth region and the gingival region are identified from the two-dimensional image 601 of the diagnosis model (hereinafter, referred to as a target diagnosis model) molded after the shape of the oral cavity of the target subject. In this case, the electronic device 100 may access the two-dimensional image 601 of the target diagnosis model received from the three-dimensional scanner 200, and input the two-dimensional image 601 of the target diagnosis model into the correlation model 500 so as to derive data in which the tooth region and the gingival region are identified. The data in which the tooth region and the gingival region are identified may represent, for example, an image in which the tooth region 603a and the gingival region 603b are masked on the input two-dimensional image 601 of the target diagnosis model. The data in which the tooth region and the gingival region are identified may represent, for example, metadata indicating positions of the tooth region and the gingival region on the input two-dimensional image 601 of the target diagnosis model.

The electronic device 100 according to various embodiments may receive the two-dimensional image 601 of the target oral cavity from the three-dimensional scanner 200 in real time while the user scans the target oral cavity through the three-dimensional scanner 200. In this case, in response to reception of the two-dimensional image 601 of the target oral cavity, the electronic device 100 may input the two-dimensional image 601 of the target oral cavity to the correlation model 500 to acquire data in which the tooth region and gingival region are identified. In addition, when the electronic device 100 sequentially receives multiple two-dimensional images 601 of the target oral cavity from the three-dimensional scanner 200, the data in which the tooth region and the gingival region are identified for each of the multiple two-dimensional images for the target oral cavity may be sequentially acquired.

The electronic device 100 according to another embodiment may receive the two-dimensional image 601 of the target diagnosis model from the three-dimensional scanner 200 in real time while the user scans the target diagnosis model through the three-dimensional scanner 200. In this case, in response to reception of the two-dimensional image 601 of the target diagnosis model, the electronic device 100 may input the two-dimensional image 601 of the target diagnosis model to the correlation model 500 to acquire data in which the tooth region and gingival region are identified.

FIG. 7 is an operational flowchart of an electronic device 100 according to various embodiments of the present disclosure. Referring to the operational flowchart 700, the at least one processor 101 of the electronic device 100 according to various embodiments may access the two-dimensional image for the target oral cavity or the target diagnosis model received from the three-dimensional scanner 200 through the communication circuit 105 in operation 710. For example, the two-dimensional image for the target oral cavity or the target diagnosis model received from the three-dimensional scanner 200 may be stored in the at least one memory 103, and the at least one processor 101 may access the two-dimensional image for the target oral cavity or the target diagnosis model stored in the at least one memory.

In operation 720, the at least one processor 101 according to various embodiments may identify the tooth region and the gingival region from the two-dimensional image for the target oral cavity or the target diagnosis model by using a correlation model (e.g., the correlation model 500 in FIG. 5) constructed through a machine learning process. The at least one processor 101 may input the two-dimensional image for the target oral cavity or the target diagnosis model to the constructed correlation model and acquire, from the correlation model, data capable of identifying the tooth region and the gingival region on the two-dimensional image for the target oral cavity or the target diagnosis model. The data capable of identifying the tooth region and the gingival region on the two-dimensional image for the target oral cavity or the target diagnosis model may include, for example, an image in which each of the tooth region and the gingival region is masked in the tooth region and the gingival region.

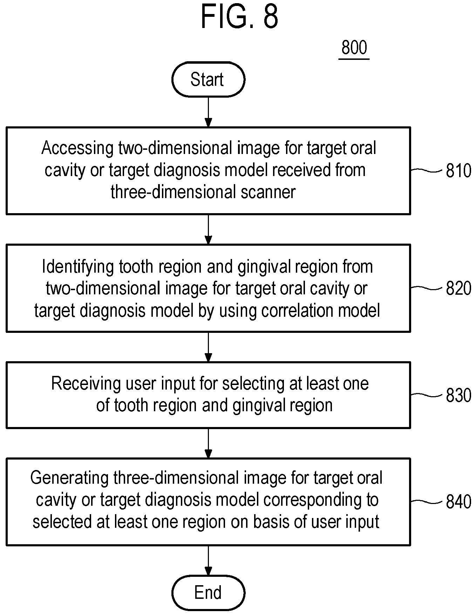

FIG. 8 is an operational flowchart of an electronic device 100 according to various embodiments of the present disclosure. A description overlapping the description in FIG. 7 will be omitted. Referring to the operational flowchart 800, the at least one processor 101 of the electronic device 100 according to various embodiments may access the two-dimensional image for the target oral cavity or the target diagnosis model received from the three-dimensional scanner 200 through the communication circuit 105 in operation 810. In operation 820, the at least one processor 101 according to various embodiments may identify the tooth region and the gingival region from the two-dimensional image for the target oral cavity or the target diagnosis model by using a correlation model (e.g., the correlation model 500 in FIG. 5) constructed through a machine learning process. The at least one processor 101 may access multiple two-dimensional images for the target oral cavity or the target diagnosis model sequentially received from the three-dimensional scanner 200 and identify the tooth region and the gingival region from each of the multiple two-dimensional images for the target oral cavity or the target diagnosis model by using the correlation model.

The at least one processor 101 according to various embodiments may receive a user input for selecting at least one of the tooth region and the gingival region through the input device 109 in operation 830. The user may select the entire region (including both the tooth region and the gingival region) among the tooth region and the gingival region, select only the tooth region, select only the tooth region and a portion of the gingival region, or select only the gingival region.

According to an embodiment, the at least one processor may display a filtering user interface (UI) for displaying a predetermined region through the display 107. The filtering UI may include an icon for displaying the entire region (including both the tooth region and the gingival region), an icon for displaying only the tooth region, and an icon for displaying only the gingival region. Furthermore, the filtering UI may further include an icon for displaying the tooth region and a portion of the gingival region.

According to an embodiment, the at least one processor 101 may display a two-dimensional image for the target oral cavity, in which the tooth region and the gingival region are identified, through the display 107. For example, the at least one processor 101 may display the tooth region in red and the gingival region in blue on the two-dimensional image for the target oral cavity or the target diagnostic model. In this case, regions (e.g., a soft tissue region) other than the tooth region and the gingival region may not be displayed in a specific color. The user may select at least one of the tooth region and the gingival region of the two-dimensional image displayed through the input device 109.

In the present figure, it is described that after operation 820 of identifying the tooth region and the gingival region from the two-dimensional image for the target oral cavity or the target diagnosis model, operation 830 of receiving a user input for selecting at least one of the tooth region and the gingival region is performed, but the present disclosure is not limited thereto, and operation 830 of receiving a user input for selecting at least one of the tooth region and the gingival region may be performed before operation 810 of accessing the two-dimensional image for the target oral cavity or the target diagnosis model received from the three-dimensional scanner, or before operation 820 of identifying the tooth region and the gingival region.

The at least one processor 101 according to various embodiments may generate, in operation 840, a three-dimensional image for the target oral cavity or the target diagnosis model corresponding to the selected at least one region in response to the user input. According to an embodiment, when the user selects only the tooth region, the at least one processor 101 may configure the tooth region as a reconstruction region, and generate a three-dimensional image for the target oral cavity or the target diagnosis model corresponding to the configured reconstruction region.

Since the user (10) scans the inside of the oral cavity of the subject 20 while holding the three-dimensional scanner 200 in his or her hand, scan data may not be acquired for some regions (e.g., narrow regions between teeth). In this case, when generating a three-dimensional image (hereinafter, referred to as a three-dimensional raw image) of the target oral cavity by using multiple two-dimensional images for the target oral cavity received through the three-dimensional scanner 200, the three-dimensional raw image for the target oral cavity may show some regions as empty regions. Accordingly, the electronic device 100 may generate a reconstructed three-dimensional image for the target oral cavity by performing various algorithms for filling in empty regions of the three-dimensional raw image for the target oral cavity and reconfiguring data.

According to an embodiment, a Poisson Algorithm may be used to fill in the empty region of the three-dimensional raw image for the target oral cavity. In this case, the electronic device 100 may convert each of the multiple two-dimensional images for the oral cavity of the target subject into a set of multiple points having three-dimensional coordinate values. For example, the electronic device 100 may convert each of the multiple two-dimensional images into a point cloud, which is a set of data points having three-dimensional coordinate values. The point cloud may be stored as raw data for the target oral cavity. The electronic device 100 may generate (reconstruct) a three-dimensional image for the target oral cavity by aligning the point cloud using the Poisson algorithm. According to the present embodiment, the electronic device 100 may identify data points corresponding to the tooth region selected as the reconstruction region from the point cloud, and apply the Poisson algorithm only to data points corresponding to the tooth region to acquire the three-dimensional image for the target oral cavity. For example, the electronic device 100 may reconfigure multiple data points corresponding to the tooth region and convert the same into a closed three-dimensional surface to generate the three-dimensional image for the target oral cavity corresponding to the tooth region. According to the present embodiment, the generated three-dimensional image for the target oral cavity may not include regions (e.g., the gingival region and soft tissue regions) other than the tooth region. In this case, since the Poisson algorithm is performed only on the tooth region, there is no need to perform the Poisson algorithm on the entire region of the target oral cavity, thereby reducing the amount of data required for generating a three-dimensional image, and time and resources required for generating a three-dimensional image.

According to an embodiment, an interpolation method may be used to fill in the empty region of the three-dimensional raw image for the target oral cavity. For example, the electronic device 100 may generate a three-dimensional image for the target oral cavity corresponding to the tooth region by filling in between multiple points included in the tooth region configured as the reconstruction region by using the interpolation method. For example, the at least one processor 101 may convert only the tooth region of the two-dimensional image for the target oral cavity into a set of multiple points having three-dimensional coordinate values. The at least one processor 101 may fill in between multiple points by estimating an arbitrary spatial position value between multiple points and adding a new point by using the interpolation method. In this manner, the three-dimensional image for the target oral cavity corresponding to the tooth region may be generated.

According to another embodiment, when the user selects only the gingival region, the at least one processor 101 may configure the gingival region as a reconstruction region, and generate a three-dimensional image for the target oral cavity or the target diagnosis model corresponding to the gingival region configured as the reconstruction region by using various algorithms. According to still another embodiment, when the user selects the entire region (including both tooth region and the gingival region), the at least one processor 101 may configure the entire region as a reconstruction region, and generate a three-dimensional image for the target oral cavity or the target diagnosis model corresponding to the entire region by using various algorithms.

According to various embodiments, the at least one processor 101 may receive a user input for selecting at least one tooth of interest from among teeth included in the tooth region through the input device 109. For example, when it is desired to acquire information on only a front tooth, the user may select the front tooth as the tooth of interest through the input device. In this case, the at least one processor 101 may generate a three-dimensional image for the target oral cavity or the target diagnosis model corresponding to the selected tooth of interest in response to the user input. The at least one processor 101 may configure a region corresponding to the selected tooth of interest as a reconstruction region and generate a three-dimensional image for the target oral cavity or the target diagnosis model corresponding to the configured reconstruction region by using various algorithms. According to the present embodiment, teeth other than the tooth of interest may not be included in the three-dimensional image.

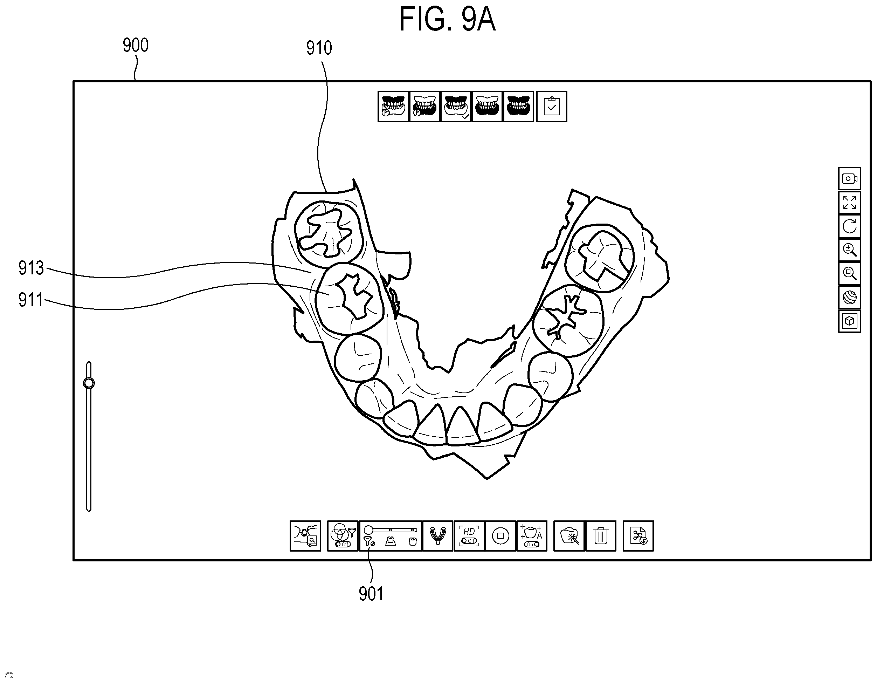

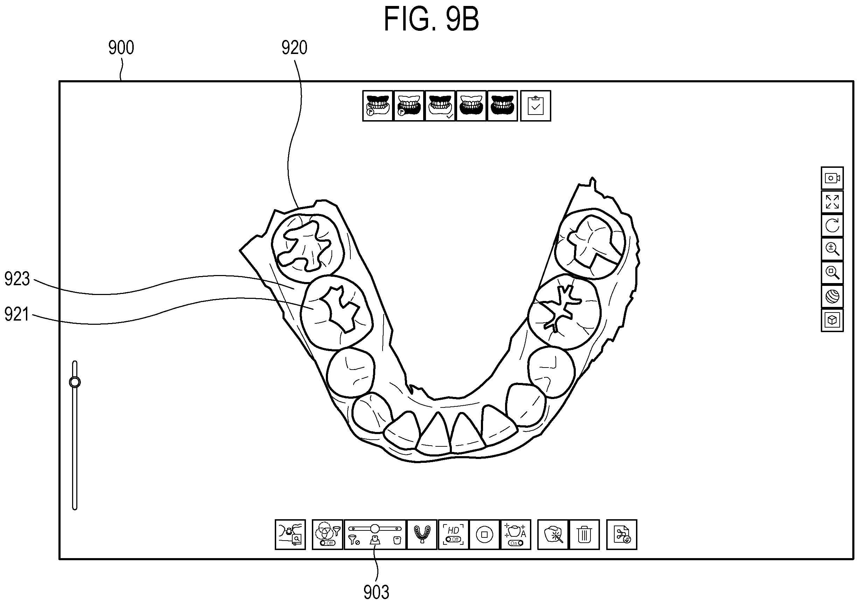

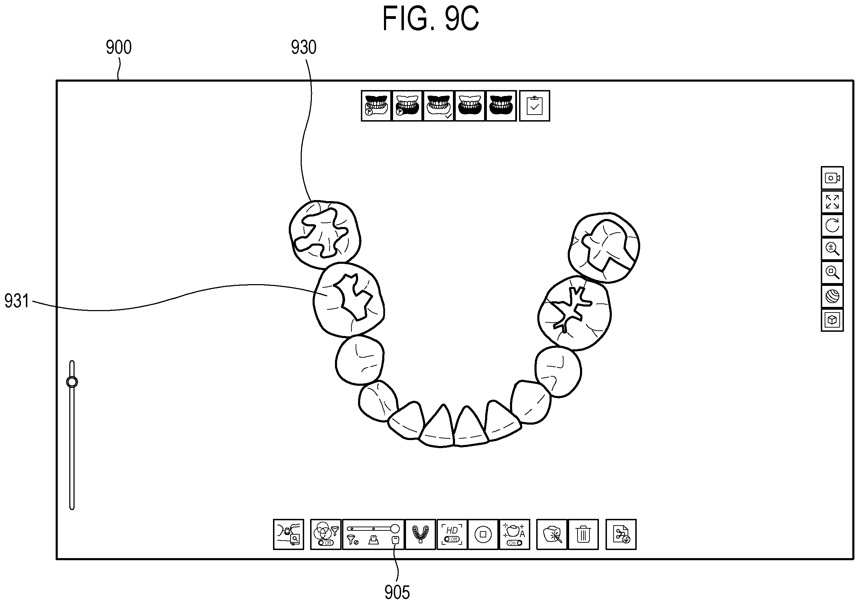

FIGS. 9A to 9C are views illustrating a three-dimensional image for a target oral cavity according to various embodiments of the present disclosure. FIG. 9A shows a three-dimensional image 910 of the target oral cavity generated when the entire region (including the tooth region and the gingival region) is configured as the reconstruction region. FIG. 9B shows a three-dimensional image 920 of the target oral cavity generated when the tooth region and a portion of the gingival region are configured as the reconstruction region. FIG. 9C shows a three-dimensional image 930 of the target oral cavity generated when the tooth region is configured as the reconstruction region. The electronic device 100 may display a three-dimensional image 910, 920, or 930 of the target oral cavity through an execution screen 900 of a predetermined application program. FIGS. 9A to 9C show a three-dimensional image for a lower jaw of the oral cavity of the subject for convenience of explanation, but the present disclosure is not limited thereto, and a three-dimensional image for an upper jaw of the subject or a three-dimensional image including both the upper and lower jaws of the subject may be generated. According to another embodiment, the electronic device 100 may generate and show a three-dimensional image for the target diagnosis model, but hereinafter, for convenience of explanation, it will be described that a three-dimensional image for the target oral cavity is generated and shown.

Referring to FIG. 9A, the user may select, through the input device 109, an icon 901 for displaying the entire region (including both the tooth region and the gingival region) from the filtering UI for displaying a predetermined region. In this case, the electronic device 100 may configure the entire region of the oral cavity of the target subject (hereinafter, referred to as the target oral cavity) as the reconstruction region, and generate a three-dimensional image 910 of the target oral cavity corresponding to the configured reconstruction region.

For example, when the user scans the inside of the oral cavity of the target subject (hereinafter, referred to as a target oral cavity) using the three-dimensional scanner 200, the electronic device 100 may receive multiple two-dimensional images for the target oral cavity from the three-dimensional scanner 200. The electronic device 100 may convert each of the multiple two-dimensional images for the target oral cavity received from the three-dimensional scanner 200 into a set (e.g., a point cloud) of multiple points having three-dimensional coordinate values. That is, since the icon 901 for displaying the entire region is selected, the electronic device 100 may convert the entire region included in each of the multiple two-dimensional images into a set of multiple points. The electronic device 100 may generate (reconstruct) the three-dimensional image 910 of the target oral cavity by performing the Poisson algorithm or the interpolation method on a set of multiple points based on the multiple two-dimensional images. In this case, the generated three-dimensional image 910 of the target oral cavity may be a three-dimensional image for the entire region (including the tooth region 911 and the gingival region 913) of the target oral cavity. The electronic device 100 may display the three-dimensional image 910 of the target oral cavity corresponding to the entire region through the execution screen 900 of a predetermined application program.

Referring to FIG. 9B, the user may select, through the input device 109, an icon 903 for displaying the tooth region and a portion of the gingival region from the filtering UI for displaying a predetermined region. In this case, the electronic device 100 may configure, as the reconstruction region, the tooth region included in the target oral cavity and a partial gingival region within a predetermined distance (e.g., 50 pixels or 1 cm) from the tooth region, and generate a three-dimensional image 920 of the target oral cavity corresponding to the configured reconstruction region.

For example, when the user scans the inside of the oral cavity of the target subject (hereinafter, referred to as a target oral cavity) using the three-dimensional scanner 200, the electronic device 100 may receive multiple two-dimensional images for the target oral cavity from the three-dimensional scanner 200. The electronic device 100 may access the multiple two-dimensional images for the target oral cavity received from the three-dimensional scanner 200 and identify the tooth region from each of the multiple two-dimensional images for the target oral cavity by using a preconstructed correlation model (e.g., the correlation model 500 in FIG. 5). The electronic device 100 may configure the identified tooth region and a partial gingival region within a predetermined distance from the tooth region as the reconstruction region. The electronic device 100 may convert the configured reconstruction region (the tooth region and the partial gingival region within a predetermined distance from the tooth region) into a set of multiple points having three-dimensional coordinate values. For example, the electronic device 100 may merges only multiple points corresponding to the configured reconstruction region by using the Poisson algorithm to generate the three-dimensional image 920 of the target oral cavity corresponding to the tooth region 921 and the partial gingival region 923. In this case, the generated three-dimensional image 920 may not include regions other than the tooth region 921 and the partial gingival region 923 configured as the reconstruction region. The electronic device 100 may display the three-dimensional image 920 of the target oral cavity corresponding to the tooth region 921 and the partial gingival region 923 through the execution screen 900 of a predetermined application program.

According to the present embodiment, since only the selected tooth region 921 and the partial gingival region 923 are configured as the reconstruction region, resources used in the process of reconstructing the three-dimensional image 920 of the target oral cavity may be significantly reduced and the time required to generate the three-dimensional image 920 of the target oral cavity may be significantly reduced. In addition, since the user may acquire the three-dimensional image 920 corresponding to a desired region, user convenience may be enhanced.

Referring to FIG. 9C, the user may select, through the input device 109, an icon 905 for displaying only the tooth region from the filtering UI for displaying a predetermined region. In this case, the electronic device 100 may configure only the tooth region included in the oral cavity as the reconstruction region, and generate a three-dimensional image 930 of the target oral cavity corresponding to the configured reconstruction region.

For example, the electronic device 100 may receive multiple two-dimensional images for the target oral cavity from the three-dimensional scanner 200. The electronic device 100 may access the multiple two-dimensional images for the target oral cavity received from the three-dimensional scanner 200 and identify the tooth region from each of the multiple two-dimensional images for the target oral cavity by using the preconstructed correlation model. The electronic device 100 may configure the identified tooth region as the reconstruction region. For example, the electronic device 100 may merge multiple points corresponding to the configured reconstruction region by using the Poisson algorithm to generate the three-dimensional image 930 of the target oral cavity corresponding to the tooth region 931. In this case, the generated three-dimensional image 930 may not include regions other than the tooth region 931 configured as the reconstruction region. The electronic device 100 may display the three-dimensional image 930 of the target oral cavity corresponding to the tooth region 931 through the execution screen 900 of a predetermined application program.

According to the present embodiment, since only the selected tooth region 931 is configured as the reconstruction region, resources used in the process of reconstructing the three-dimensional image 930 of the target oral cavity may be significantly reduced and the time required to generate the three-dimensional image 930 of the target oral cavity may be significantly reduced. In addition, since the user may acquire the three-dimensional image 930 corresponding to a desired region, user convenience may be enhanced.

According to another embodiment, the electronic device 100 may configure only the gingival region included in the oral cavity as the reconstruction region, and generate a three-dimensional image (not shown) of the target oral cavity corresponding to the gingival region configured as the reconstruction region.

Accordingly, the user may select a predetermined region for constructing a three-dimensional image as needed, and acquire the three-dimensional image for the target oral cavity corresponding to the desired region.

Further, although process operations, method operations, algorithms or the like shown in the flowchart may be described in a sequential order, such processes, methods and algorithms may be configured to work in any suitable order. In other words, any sequence or order of operations that may be described in the present disclosure does not, in and of itself, indicate a requirement that the operations be performed in that order. Further, some operations may be performed simultaneously in other embodiments despite being described or implied as occurring non-simultaneously. Moreover, the illustration of a process by its depiction in a drawing does not imply that the illustrated process is exclusive of other variations and modifications thereto, does not imply that the illustrated process or any of its operations are necessary to one or more embodiments of the present disclosure, and does not imply that the illustrated process is preferred.

Although the aforementioned method is described through a predetermined embodiment, the method may also be implemented as computer readable code on a computer-readable recording medium. The computer-readable recording medium includes all types of recording devices in which data that may be read by a computer system is stored. Examples of the computer-readable recording medium include a ROM, a RAM, a CD-ROM, a magnetic tape, a floppy disk, optical data storage, and the like. Furthermore, the computer-readable recording medium may be distributed to computer systems which are connected through a network so that computer-readable codes may be stored and executed in a distributed manner. In addition, functional programs, codes, and code segments for implementing the embodiments may be easily inferred by programmers in the technical field to which the present disclosure pertains.

Although the technical spirit of the present disclosure has been described by the examples described in some embodiments and illustrated in the accompanying drawings, it should be noted that various substitutions, modifications, and changes can be made without departing from the scope of the present disclosure which can be understood by those skilled in the art to which the present disclosure pertains. In addition, it should be noted that that such substitutions, modifications and changes are intended to fall within the scope of the appended claims.

## Claims

1. An electronic device comprising:
a communication circuit communicatively connected to a three-dimensional scanner;
at least one memory configured to store a correlation model constructed by modeling a correlation between a two-dimensional image set regarding oral cavities of subjects and a data set in which a tooth region and a gingival region are identified in each image of the two-dimensional image set according to a machine learning algorithm; and
at least one processor,
wherein the at least one processor is configured to:
access a two-dimensional image regarding a target oral cavity or target diagnosis model received from the three-dimensional scanner through the communication circuit; and
use the correlation model to identify a tooth region and a gingival region from the two-dimensional image regarding the target oral cavity or target diagnostic model.

2. The electronic device of claim 1, wherein the data set comprises multiple two-dimensional images, and each of the multiple two-dimensional images is an image in which each of a tooth region and a gingival region is masked in each image of the two-dimensional image set.

3. The electronic device of claim 1, further comprising a display,
wherein the at least one processor is configured to display, through the display, a two-dimensional image regarding the target oral cavity or target diagnosis model, in which the tooth region and the gingival region are identified.

4. The electronic device of claim 1, further comprising an input device,
wherein the at least one processor is configured to:
receive, through the input device, a user input for selecting at least one of the tooth region and the gingival region; and
generate, in response to the user input, a three-dimensional image regarding the target oral cavity or target diagnosis model corresponding to the selected at least one region.

5. The electronic device of claim 4, wherein the at least one processor is configured to:
set the selected at least one region as a reconstruction region; and
generate a three-dimensional image regarding the target oral cavity or target diagnosis model by using a Poisson algorithm to connect multiple points included in the set reconstruction region to each other.

6. The electronic device of claim 4, wherein the at least one processor is configured to:
set the selected at least one region as a reconstruction region; and
generate a three-dimensional image regarding the target oral cavity or target diagnosis model by using an interpolation method to fill a gap between multiple points included in the set reconstruction region.

7. The electronic device of claim 4, wherein the at least one processor is configured to transmit the generated three-dimensional image regarding the target oral cavity or target diagnosis model to a cloud server.

8. The electronic device of claim 1, wherein the correlation model is a correlation model machine-trained to extract at least one feature of texture, density, color, tooth shape, gingival shape, and interoral cavity shape of the tooth region and the gingival region identified in each image of the two-dimensional image set, and derive a correlation between the two-dimensional image set and the data set by using the extracted at least one feature.

9. The electronic device of claim 1, wherein the machine learning algorithm is one of a deep neural network, a recurrent neural network, a convolutional neural network, a machine learning model for classification-regression analysis, or a reinforcement learning model.

10. An image processing method of an electronic device, the method comprising:
accessing a two-dimensional image regarding a target oral cavity or target diagnosis model received from a three-dimensional scanner; and
using a correlation model to identify a tooth region and a gingival region from the two-dimensional image regarding the target oral cavity or target diagnostic model,
wherein the correlation model is a correlation model constructed by modeling a correlation between a two-dimensional image set regarding oral cavities of subjects and a data set in which a tooth region and a gingival region are identified in each image of the two-dimensional image set according to a machine learning algorithm.

11. The method of claim 10, wherein the data set comprises multiple two-dimensional images, and each of the multiple two-dimensional images is an image in which each of a tooth region and a gingival region is masked in each image of the two-dimensional image set.

12. The method of claim 10, further comprising displaying, through a display, a two-dimensional image regarding the target oral cavity or target diagnosis model, in which the tooth region and the gingival region are identified.

13. The method of claim 10, further comprising:
receiving, through an input device, a user input for selecting at least one of the tooth region and the gingival region; and
generating, in response to the user input, a three-dimensional image regarding the target oral cavity or target diagnosis model corresponding to the selected at least one region.

14. The method of claim 13, wherein the generating of the three-dimensional image comprises: setting the selected at least one region as a reconstruction region; and
generating a three-dimensional image regarding the target oral cavity or target diagnosis model by using a Poisson algorithm to connect multiple points included in the configured reconstruction region to each other.

15. The method of claim 13, wherein the generating of the three-dimensional image comprises: setting the selected at least one region as a reconstruction region; and
generating a three-dimensional image regarding the target oral cavity or target diagnosis model by using an interpolation method to fill a gap between multiple points included in the set reconstruction region.

16. The method of claim 13, further comprising transmitting the three-dimensional image regarding the target oral cavity or target diagnosis model to a cloud server.

17. The method of claim 10, wherein the correlation model is a correlation model machine-trained to extract at least one feature of texture, density, color, tooth shape, gingival shape, and interoral cavity shape of the tooth region and the gingival region identified in each image of the two-dimensional image set, and derive a correlation between the two-dimensional image set and the data set by using the extracted at least one feature.

18. The method of claim 10, wherein the machine learning algorithm is one of a deep neural network, a recurrent neural network, a convolutional neural network, a machine learning model for classification-regression analysis, or a reinforcement learning model.

19. An electronic device for training a machine learning model used for identifying a tooth region and a gingival region, the electronic device comprising:
at least one memory configured to store a two-dimensional image set regarding oral cavities of subjects or oral cavity diagnosis model and a data set in which a tooth region and a gingival region are identified in each image of the two-dimensional image set; and
at least one processor,
wherein the at least one processor is configured to use the two-dimensional image set of the oral cavities of the subjects as input data and the data set in which the tooth region and the gingival region are identified in each image of the two-dimensional image set as output data, so as to train a machine learning model.

20. The electronic device of claim 19, wherein the data set comprises multiple two-dimensional images, and each of the multiple two-dimensional images is an image in which each of a tooth region and a gingival region is masked in each image of the two-dimensional image set.
